# FASCICULE DE BREVET EUROPEEN

(11) **EP 3 083 410 B1**
(45) Date de publication et mention de la délivrance du brevet: **01.05.2019**
(21) Numéro de dépôt: 14828261.9
(22) Date de dépôt: 19.12.2014
(51) Int. Cl.: B65B 11/48, B65B 11/50, B65B 55/08, C08J 7/04, C12M 1/00, C12N 1/04, B32B 27/00, B65D 65/42, B65D 77/04

(54) **UTILISATION DE FILM POLYMERE POUR L'EMBALLAGE DE MILIEU DE CULTURE**
NÜTZUNG VON EINEM POLYMERFILM FÜR DIE VERPACKUNG EINES NÄHRUNGSBODENS
USE OF A POLYMERIC FILM FOR THE PACKAGING OF A CULTURE MEDIUM

(30) Priorité: 20.12.2013 FR 1363241
(43) Date de publication de la demande: 26.10.2016
(73) Titulaire: bioMérieux, 69280 Marcy-L'Etoile (FR)
(72) Inventeur: TETART, Bruno, F-69290 Craponne (FR); SIMON, Nathalie, F-69290 Grézieu La Varenne (FR)
(86) Numéro de dépôt international: PCT/FR2014/053461
(87) Numéro de publication internationale: WO 2015/092325

(56) Documents cités:
- FR-A1- 2 913 021
- JP-A- H0 698 675

## Description

De nombreux films polymères, aptes à être utilisés pour l'emballage des produits sont présents sur le marché. On peut citer en particulier les films à usage alimentaire tels que les films en polyamide (PA), en polyéthylène téréphtalate (PET) ou en polychlorure de vynile (PVC).

Lorsque l'on s'intéresse plus particulièrement au domaine du diagnostic in vitro, qui est le domaine d'activité de la demanderesse, et en particulier aux films utilisés pour l'ensachage des milieux de culture, on constate que les matériaux habituellement utilisés sont les matériaux présentant une propriété barrière faible caractérisée par une perméabilité élevée à la vapeur d'eau (>120g/m² x 24 heures). Un tel matériau est par exemple la cellophane. Ce matériau a pour avantage de permettre à l'eau contenue dans les milieux de culture gélosés prêts à l'emploi de s'évaporer et de traverser le film. Ceci empêche alors une condensation trop importante à l'intérieur du sac constitué du film en cellophane. Par contre, l'inconvénient principal est que, la vapeur d'eau traversant le film, le taux d'humidité à l'intérieur est très bas, entraînant un dessèchement plus important et donc prématuré du milieu de culture. La durée de conservation du produit s'en trouve donc affectée.

D'autres matériaux également utilisés pour l'ensachage des milieux de culture présentent, quant à eux, une propriété barrière élevée caractérisée par une perméabilité faible à la vapeur d'eau (<5g/m² x 24 heures). Cette faible perméabilité à la vapeur d'eau ne permet pas d'évacuer la condensation importante qui se forme dans les boîtes de milieux gélosés prêts à l'emploi, notamment après que les milieux ont été coulés. Il s'ensuit que cette eau demeure dans le sac jusqu'à l'ouverture de celui-ci par l'utilisateur final, générant éclaboussures et souillures ; ce qui est rédhibitoire. De tels produits sont par exemple les polyoléfines, tels que les polypropylènes (PP) ou polyéthylène (PE). Les polyoléfines sont largement utilisés comme matériau d'emballage. Toutefois, les procédés d'obtention de tels matériaux font que ces derniers présentent une très faible perméabilité à la vapeur d'eau. Par ailleurs, on trouve également des matériaux comportant deux films complexés, tels que des films PA + PE, destinés à accentuer leur propriété barrière à la vapeur d'eau. Ainsi des matériaux de ce type présentent des valeurs de perméabilité à la vapeur d'eau, inférieures à une dizaine de grammes/m² x 24 heures. Des solutions connues pour limiter la quantité d'eau demeurant dans le sac jusqu'à l'ouverture consiste en l'utilisation de dessicants tel que des gels de silice sous forme de sachets. Une telle méthode oblige cependant à ajouter une quantité de dessicant dans chacun des sac, entrainant ainsi des surcouts de fabrication et une quantité de déchets importante.

Enfin, d'autres matériaux également utilisés pour l'ensachage des milieux de culture comportent une monocouche uniquement constitué de PA non orienté, de type "cast" et d'autre part, une quantité de PVC et/ou polychlorure de vinylidène (PVDC) utilisé comme constituant de base d'une couche d'enduit de scellage du film. L'intérêt de cette technique est de pouvoir moduler la perméabilité à la vapeur d'eau, en faisant varier la quantité d'enduit déposé sur le film PA. Des matériaux de ce type présentent une perméabilité à la vapeur d'eau comprise entre 35g/m²x24 heures et 110g/m²x24 heures. Cependant les procédés de réalisation de ces films ne permettent pas de maîtriser la quantité d'enduit déposé sur la surface du film. De ce fait, le film obtenu présente une gamme de perméabilité à la vapeur d'eau très variable pour un même lot de fabrication. Par conséquent, l'utilisation de ce type de film pour la réalisation d'emballages de milieu de culture ne permet pas de garantir une durée de péremption précise.

Le document FR 2 913 021 décrit l'utilisation d'un film polymère, comprenant une feuille de polyamide recouverte sur l'une de ces faces d'une enduite thermoscellant, pour l'emballage de boites de Pétri comprenant un milieu de culture de microorganismes sous forme gélosée. Le document JP H06 98674 décrit l'utilisation d'un film de polystyrène pour l'ensachage de champignon frais.

Il s'ensuit que les sociétés productrices de milieux de culture gélosés sont toujours dans l'attente d'un emballage apte à conserver lesdits milieux de culture, dans des conditions optimales, à savoir dans un environnement suffisamment riche en vapeur d'eau pour éviter leur dessèchement prématuré, mais également suffisamment pauvre pour éviter une condensation trop importante dans le sac, notamment à température ambiante, et ce de manière prédictible et peu variable dans le temps. De tels films devraient permettre de limiter la cinétique de perte de poids de la gélose ainsi que le risque de déshydratation des milieux de culture sans dégrader le niveau d'exsudation de l'emballage. Ces propriétés doivent par ailleurs être combinées avec un rendu visuel conforme aux attentes des clients, notamment en terme de transparence ainsi qu'une résistance à l'étirement satisfaisante.

Il est du mérite des inventeurs d'avoir mis en évidence l'utilisation d'un film polymère, pour emballer au moins une boite de Pétri contenant un milieu de culture de microorganismes sous forme gélosée selon la revendication 1. L'utilisation de ces matériaux particuliers permettant une meilleure maitrise de la durée de conservation et ce quelle que soit les conditions de stockage et de transport. Le stockage et transport à température ambiante étant possible.

Ainsi, un premier objectif de la présente invention est de proposer une utilisation d'un film possédant des propriétés physiques, particulièrement en terme de capacité barrière à la vapeur d'eau, aptes à permettre une durée de conservation améliorée et moins variable des milieux de cultures de microorganismes sous une atmosphère à taux d'humidité contrôlé.

Un deuxième objectif de la présente invention est de proposer une utilisation d'un film permettant de réduire la quantité de gélose présente dans le milieu de culture de microorganismes.

Un troisième objectif de la présente invention est de proposer une utilisation d'un film souple et de faible épaisseur et présentant par ailleurs un cout de revient limité.

Un quatrième objectif de la présente invention est de proposer une utilisation d'un film facilement scellable pour la réalisation d'un sachet d'emballage pour milieux de culture de microorganismes.

Un cinquième objectif de la présente invention est de proposer une utilisation d'un film apte à répondre aux standards en terme de rendu esthétique, particulièrement en terme de transparence et de toucher.

Un sixième objectif de la présente invention est de proposer une utilisation d'un film apte à répondre aux standards en terme de résistance à la rupture et de déformation élastique.

Un septième objectif de la présente invention est de proposer une utilisation d'un film possédant des propriétés physiques, particulièrement en terme de capacité barrière à la vapeur d'eau, aptes à permettre une durée de conservation améliorée quelles que soient les conditions de température.

Un huitième objectif de la présente invention est de proposer une utilisation d'un film possédant des propriétés physiques, particulièrement en terme de capacité barrière à la vapeur d'eau, aptes à permettre une diminution de la cinétique de perte de poids gélose et à aptes à permettre une meilleure stabilité de la cinétique de perte de poids gélose dans le temps, et ce quelles que soient les conditions de température.

Un autre objectif de la présente invention est de proposer une utilisation d'un film permettant la réalisation d'un emballage réutilisable et/ou refermable.

Ces objectifs parmi d'autres, sont atteints par la présente invention qui concerne en premier lieu l'utilisation d'un film polymère pour emballer au moins une boîte de Pétri contenant un milieu de culture de microorganismes sous forme gélosée selon la revendication 1.

Par film polymère, on entend un matériau comprenant au moins une couche d'un matériau polymère , sans limitation de taille, tel que le polystyrène. De tels films peuvent être réalisés par extrusion ou coextrusion afin d'obtenir un film comprenant plusieurs couches, chacune ayant leur qualités propres.

Les différentes mesures de perméabilité à la vapeur d'eau des films polymères décrits dans la présente invention sont déterminées à 38°C et 90% d'humidité relative selon la norme NF ISO 2528 (09/2001).

On entend par milieu de culture, un milieu comprenant tous les constituants nécessaires à la survie et/ou à la croissance de microorganismes, déposé sur un support. En pratique, l'homme du métier choisira le milieu de culture en fonction des microorganismes cibles, selon des critères parfaitement connus et à la portée de cet homme de l'art. Un milieu de culture peut se présenter sous une forme déshydratée ou gélosée. Dans le cas de la forme gélosée, le milieu de culture est contenu dans une boîte de Petri. Les boîte de Pétri sont généralement constituées d'un fond, dans lequel est coulé à chaud le milieu de culture gélosé, également appelé gélose et d'un couvercle. Les parties extérieures du fond et du couvercle coopérant afin de pouvoir empiler plusieurs boîtes de Petri. Généralement, des piles de dix boîtes sont réalisées pour le conditionnement et le transport.

On entend par couche thermoscellante un couche polymère susceptible de solidariser au moins partiellement, sous l'effet de la chaleur, deux bord superposés sur au moins un côté d'un film. De façon préférentielle, l'étape de solidarisation est effectuée par thermoscellage à une température comprise entre 100 et 170 °C. Des exemples de matériaux pouvant constituer une telle couche sont le Polyéthylène, le Polypropylène ou encore le Polychlorure de Vinyle.

Un avantage de l'utilisation d'un film polymère, pour emballer au moins un milieu de culture de microorganismes, comprenant au moins une couche de polystyrène et au moins une couche thermoscellante est d'obtenir un emballage pour milieu de culture réutilisable et/ou refermable, celui-ci étant non-étirable. Contrairement à l'utilisation de films étirables particulièrement ductiles tel que des films en Polychlorure de vinyle, les emballages ainsi formés par thermoscellage peuvent continuer à contenir ou maintenir un ou plusieurs milieux de culture une fois ouverts. Un opérateur peut ainsi déplacer aisément l'emballage et son contenu sans risquer de faire tomber un ou plusieurs milieux de cultures. Les films étirables sont connus pour se déchirer à l' ouverture de l'emballage, empêchant ainsi toute manipulation ultérieure de celui-ci. De plus les milieux contenus par ce type d'emballage risquent de ne plus être maintenus convenablement en pile après ouverture.

Avantageusement le film utilisé selon l'invention est un film non-étirable. Plus avantageusement la couche thermoscellante du film est non-étirable afin de rendre le film utilisé selon l'invention non-étirable.

Préférentiellement, le film utilisé présente un allongement à la rupture inférieur à 250% et ce quelle que soit le sens de mesure (machine ou transverse), lui permettant d'être peu ductile. Plus préférentiellement, le film utilisé présente un allongement à la rupture inférieur à 100% et ce quelle que soit le sens de mesure (machine ou transverse), lui permettant d'être très peu ductile. Encore plus préférentiellement, le film utilisé présente un allongement à la rupture inférieur à 50% et ce quelle que soit le sens de mesure (machine ou transverse) afin d'assurer un meilleur maintien des milieux de culture dans l'emballage après ouverture.

Préférentiellement, le film utilisé présente une résistance au déchirement minimale de 5N/mm (ISO 6383-1) ce quelle que soit la direction de mesure (machine ou transverse). Plus préférentiellement, le film utilisé présente une résistance à la rupture minimale de 20N/mm (ISO 6383-1) et ce selon la direction de mesure machine, lui permettant d'être plus résistant.

Préférentiellement, le film utilisé présente une contrainte à la rupture minimale de 25Mpa (ISO 527) ce quelle que soit la direction de mesure (machine ou transverse) afin d'être suffisamment résistant dans une utilisation classique.

Selon une caractéristique préférentielle, le film utilisé pour emballer au moins un milieu de culture de microorganismes est transparent. Cette transparence permet notamment d'identifier par tout moyen le milieu de culture emballé sans l'ouvrir. Cette transparence permet notamment la reconnaissance de code à barres présents sur le support du milieu de culture par un lecteur de code à barre ou tout autre moyen d'imagerie. Un autre intérêt est également de pouvoir voir la bonne qualité du milieu du culture en recherchant d'éventuels défauts d'aspects et/ou contaminations du milieu de culture avant ouverture de l'emballage.

Selon l'invention, le film utilisé pour emballer au moins un milieu de culture de microorganismes comprend au moins une couche microperforée.

Par microperforée, on entend tout moyen susceptible de modifier la perméabilité à la vapeur d'eau d'une couche polymère par la réalisation de perforations de tailles comprise entre 10µm et 50µm. Le nombre et l'espacement des perforations permet également de modifier la perméabilité d'une couche polymère à la vapeur d'eau, de façon contrôlée. De façon préférentielle, les microperforations sont réalisées par un laser. De façon plus préférentielle, une seule couche de polyéthylène téréphtalate est microperforée. De façon encore plus préférentielle et dans le cas où le film comprend deux couches de polyéthylène téréphtalate, ces deux couches sont microperforées.

Selon une autre caractéristique préférentielle, le film utilisé pour emballer au moins un milieu de culture de microorganismes présente une épaisseur comprise entre 30 et 50 µm. Une épaisseur comprise entre 20 et 80 µm permet de garantir une résistance à la déchirure et un aspect visuel acceptable, permettant à l'opérateur de visualiser facilement le type de milieu de culture emballé dans le sachet formé par le film. Une épaisseur comprise entre 30 et 50 µm permet de limiter le coût d'achat et l'utilisation de matière tout en garantissant un aspect visuel acceptable, la gamme d'épaisseur pouvant être adaptée à l'aspect du milieu de culture emballé et à la résistance à la déchirure recherchée.

Un autre objet de l'invention concerne l'utilisation d'un film polymère tel que décrit précédemment pour la réalisation d'un sachet destiné à l'emballage d'au moinsune une boîte de Pétri contenant un milieu de culture de microorganismes sous forme gélosée selon la revendication 3.

Un autre objet de l'invention concerne un procédé d'emballage d'au moins un milieu de culture selon la revendication 5.

Préférentiellement, le ou les films sont préalablement stérilisés. La méthode de stérilisation peut être une irradiation par rayonnements pris dans le groupe constitué par les rayons gamma et/ou béta.

Préférentiellement, l'étape de solidarisation est une étape de thermoscellage à une température comprise entre 100 et 170 °C.

Selon une autre caractéristique préférentielle, le procédé d'emballage selon l'invention comporte en outre les étapes additionnelles consistant à :
- placer le sachet ainsi obtenu à l'intérieur d'un deuxième sachet et
- sceller ledit deuxième sachet.

Selon une autre caractéristique préférentielle, le procédé d'emballage selon l'invention comporte en outre les étapes additionnelles consistant à :
- placer le deuxième sachet ainsi obtenu à l'intérieur d'un troisième sachet et
- sceller ledit troisième sachet.

Selon une autre caractéristique préférentielle, lesdits deuxième et/ou troisième sachets du procédé d'emballage selon l'invention sont constitués d'un matériau pris dans le groupe comprenant : la cellophane, les polyoléfines, les polyamides.

Selon une autre caractéristique préférentielle, lesdits deuxième et/ou troisième sachets du procédé d'emballage selon l'invention sont constitués d'un film comprenant au moins une couche de polystyrène et au moins une couche thermoscellante, tel que le polyéthylène, ledit film présentant une perméabilité à la vapeur d'eau moyenne comprise entre 30,0 g/m²x24 heures et 140,0 g/m²x24 heures, préférentiellement entre 30,0 g/m²x24 heures et 130,0 g/m²x24 heures, plus préférentiellement entre 30,0 g/m²x24 heures et 120,0 g/m²x24 heures, encore plus préférentiellement entre 70,0 g/m²x24 heures et 120,0 g/m²x24 heures.

Alternativement lesdits deuxième et/ou troisième sachets du procédé d'emballage selon l'invention sont constitués d'un film présentant une perméabilité à la vapeur d'eau moyenne comprise entre 70,0 g/m²x24 heures et 140,0 g/m²x24 heures, préférentiellement entre 70,0 g/m²x24 heures et 130,0 g/m²x24 heures.

Une autre forme de réalisation, qui ne fait pas partie de la présente invention, concerne l'utilisation d'un film tel que décrit précédemment pour emballer au moins un milieu de culture dans un isolateur ou dans une hotte à flux d'air laminaire. L'intérêt de l'utilisation d'un tel film dans ce type d'application est de pouvoir décontaminer l'extérieur d'un emballage formé par ledit film sans risquer d'endommager le ou les milieux de cultures présents dans l'emballage ou de détruire d'éventuels microorganismes présents sur le milieu de culture pour analyse. En effet, un tel film présente une étanchéité aux principaux gaz de décontamination utilisés dans des isolateurs tel que le péroxyde d'hydrogène (H₂O₂) ou l'acide peracétique (C₂H₄O₃).

Les buts et avantages de la présente invention seront mieux compris à la lumière des exemples nullement limitatifs qui suivent, en référence au dessin.

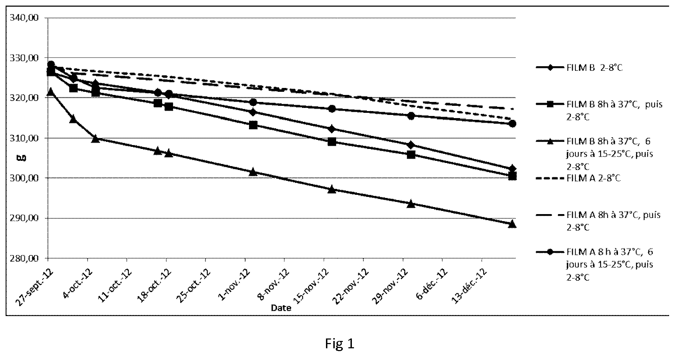

La figure 1 présente les mesures des poids des piles complètes de boites semaine par semaine pour les lots LOT 1 et LOT 2 en fonction des températures de stockage.

### Exemple :

Différents lots de boites de Pétri sont constitués. Chacun des lots contient dix milieux de cultures gélosé de type Mac Conkey fabriqués par la demanderesse.

Le premier lot, LOT 1, comprend trois piles de dix boites de Petri, chaque pile étant emballée dans un sachet formé à partir d'un film PS comprenant une couche de polystyrène, appelé FILM A d'une épaisseur de 30µm.

Le second lot, LOT 2, comprend trois piles de dix boites de Petri, chaque pile étant emballée dans un sachet formé à partir d'un film de référence comprenant une couche cellophane appelé FILM B d'une épaisseur de 30µm et d'une perméabilité à la vapeur d'eau d'environ 370g/m²x24hr.

Les coefficient de transmission de la vapeur d'eau du film A est déterminé par cinq mesures selon la norme précitée. Les résultats sont indiqués dans le tableau la suivant.

**Tableau 1a**

| Coefficient de transmission de la vapeur d'eau | | | | | | | |
|---|---|---|---|---|---|---|---|
| (g/m².24h) | | | | | | | |
| Référence | Résultats | | | | | moyenne | écart-type |
| **FILM A : PS** | 97.3 | 97.3 | 97.2 | 99.4 | 95.7 | 97.4 | 1.3 |

Les caractéristiques mécaniques du FILM A sont indiquées dans le tableau 1b suivant :

**Tableau 1b (SM : sens machine, TM : sens transversal). Les valeurs de ce tableau obéissent à une tolérance de 20%.**

| **Caractéristiques** | **Méthodes** | **valeurs** | **unités** |
|---|---|---|---|
| Epaisseur | NFT 54-101 | 30 | µm |
| Traitement Corona | | si nécessaire | |
| densité | ISO 1183 | 1,036 | |
| Contrainte à la rupture | ISO 527 | SM : 34 ST : 30 | Mpa |
| Allongement à la rupture | ISO 527 | SM : 6 ST:5 | % |
| Résistance au déchirement | ISO 6383-1 | SM : 20 ST : 5 | N/mm |
| Resistance au choc | ISO 7765-1 | < 25 - methode A | g |

Chacune des trois piles des deux lots ainsi formés est ensuite stockée durant 11 semaines selon plusieurs conditions de températures. Une première pile de chaque lot est ainsi stockée à une température comprise entre 2 et 8°C. Une seconde pile de chaque lot est stockée durant 8 heures à 37°C, puis à une température comprise entre 2 et 8°C. Un troisième pile de chaque lot est stockée durant 8 heures à 37°C, puis durant 6 jours à une température comprise entre 15°C et 25°C et enfin à une température comprise entre 2 et 8°C

Le poids total de chaque pile de chaque lot est mesuré toute les semaines afin de déterminer la cinétique de perte de poids de la gélose en fonction des conditions de stockage et d'emballage. Le suivi du poids de la gélose est un indicateur de la quantité d'eau perdue par la gélose.

Les résultats de ces mesures sont donnés dans le tableau suivant et en relation avec la figure 1.

Le tableau 2 présente le suivi du poids, en gramme, des piles de boites, pour chaque lot.

**Tableau 2**

| | 27-sept.-12 | 1-oct.-12 | 5-oct.-12 | 16-oct.-12 | 18-oct.-12 | 2-nov.-12 | 16-nov.-12 | 30-nov.-12 | 18-déc.-12 |
|---|---|---|---|---|---|---|---|---|---|
| FILM B 2-8°C | 326,44 | 324,68 | 323,63 | 321,32 | 320,64 | 316,45 | 312,26 | 308,21 | 302,28 |
| FILM B 8h à 37°C, puis 2-8°C | 326,49 | 322,38 | 321,28 | 318,60 | 317,82 | 313,29 | 309,04 | 305,84 | 300,46 |
| FILM B 8h à 37°C, 6 jours à 15-25°C, puis 2-8°C | 321,59 | 314,70 | 309,90 | 306,78 | 306,16 | 301,48 | 297,24 | 293,64 | 288,50 |
| FILM A 2-8°C | 327,84 | 327,17 | 326,66 | 325,50 | 325,22 | 322,98 | 321,01 | 318,00 | 314,73 |
| FILM A 8h à 37°C, puis 2-8°C | 328,14 | 326,16 | 325,73 | 324,52 | 324,25 | 322,34 | 320,78 | 319,06 | 317,26 |
| FILM A 8 h à 37°C, 6 jours à 15-25°C, puis 2-8°C | 328,32 | 324,97 | 322,52 | 321,28 | 321,02 | 318,90 | 317,23 | 315,52 | 313,57 |

Les tableau 2 ainsi que la figure 1 montrent ainsi une réduction de la cinétique de perte de poids de la gélose grâce à l'utilisation des films FILM A en comparaison avec un film de référence FILM B.

Les résultats sur la cinétique de perte de poids de la gélose ont été obtenus en maintenant un niveau de condensation acceptable à l'intérieur des sachets utilisant le FILM A, contrairement aux sachets utilisant des films plastiques standard.

Il est démontré une diminution de la cinétique de perte de poids de la gélose quelles que soient les conditions de stockage. L'influence de la température sur la cinétique de perte de poids gélose étant diminuée grâce à l'utilisation du FILM A en comparaison avec le film de référence FILM B utilisé pour la réalisation des sachets. En effet, toutes conditions de stockage confondues, la perte de poids de la gélose est diminuée pour le lot utilisant des films FILM A. Les piles du lot stockées à une température comprise entre 2 et 8°C dans des films FILMA présentent notamment un poids en faible décroissance au fil des semaines en comparaison avec les piles emballées dans des films FILM B. L'amélioration est encore plus notable pour les piles stockées durant 8 heures à 37°C, puis durant 6 jours à une température comprise entre 15°C et 25°C et enfin à une température comprise entre 2 et 8°C

Enfin il est également démontré une meilleure stabilité de la cinétique de perte de poids de la gélose quelles que soient les conditions de stockage, les mesures effectuées sur les lots emballés par le FILM B montrant une plus grande variation de la cinétique de perte de poids gélose dans le temps.

Ces résultats permettent ainsi d'envisager une réduction de la quantité de gélose pouvant être coulée dans une boite de Pétri en permettant néanmoins de garantir une durée de péremption semblable aux standards actuels. Inversement, des durées de conservation étendues peuvent être atteintes en utilisant des films selon l'invention en conservant une quantité de gélose coulée similaire. L'utilisation de film selon l'invention permet donc une réduction des coûts de fabrication et / ou un allongement de la durée de conservation des milieux de culture.

## Revendications

1. Utilisation d'un film polymère, pour emballer au moins une boite de Pétri contenant un milieu de culture de microorganismes sous forme gélosée, ledit film comprenant au moins une couche de polystyrène et au moins une couche thermoscellante, ledit film présentant une perméabilité à la vapeur d'eau moyenne comprise entre 30,0 g/m²x24 heures et 140,0 g/m²x24 heures, préférentiellement entre 70,0 g/m²x24 heures et 120,0 g/m²x24 heures, et dans laquelle au moins une couche de polymère est microperforée avec des perforations de taille comprise entre 10µm et 50µm.

2. Utilisation selon la revendication 1 dans laquelle le film présente une épaisseur comprise entre 30 et 50 µm.

3. Utilisation d'un film polymère, pour la réalisation d'un sachet destiné à l'emballage d'au moins une boite de Pétri contenant un milieu de culture de microorganismes sous forme gélosée, ledit film comprenant au moins une couche de polystyrène et au moins une couche thermoscellante, ledit film présentant une perméabilité à la vapeur d'eau moyenne comprise entre 30,0 glm2 x24 heures et 140,0 g/m2 x24 heures, préférentiellement entre 70,0 g/m2 x24 heures et 120,0 g/m2x24 heures, et dans laquelle au moins une couche de polymère est microperforée avec des perforations de taille comprise entre 10µm et 50 µm.

4. Procédé d'emballage d'au moins un milieu de culture, comprenant les étapes consistant à :
- placer le(s) milieu(x) de culture sur un film comprenant au moins une couche de polystyrène et au moins une couche thermoscellante, ledit film présentant une perméabilité à la vapeur d'eau moyenne comprise entre 30,0 g/m²x24 heures et 140,0 g/m²x24 heures, préférentiellement entre 70,0 g/m²x24 heures et 120,0 g/m²x24 heures, et au moins une couche de polymère dudit film étant microperforée avec des perforations de taille comprise entre 10µm et 50µm, le placement du ou des milieux de culture étant réalisé sur la couche thermoscellante dudit film;
- recouvrir le(s) milieu(x) de culture avec une portion restée libre dudit film ou avec un autre film, de sorte que les couches thermoscellantes soient en regard l'une de l'autre,
- solidariser les bords du ou des deux films, de sorte que le(s) milieu(x) de culture soit emprisonné dans le sachet ainsi formé.

5. Procédé d'emballage selon la revendication précédente dans lequel le ou les films sont préalablement stérilisée(s).

6. Procédé d'emballage selon la revendication précédente, dans lequel la méthode de stérilisation est une irradiation par rayonnements pris dans le groupe constitué par rayons gamma et/ou béta.

7. Procédé d'emballage selon l'une des revendications 4 à 6, dans lequel l'étape de solidarisation est une étape de thermoscellage à une température comprise entre 100 et 170 °C.

8. Procédé d'emballage selon l'une des revendications 4 à 7 comportant en outre les étapes additionnelles consistant à :
- placer le sachet ainsi obtenu à l'intérieur d'un deuxième sachet et
- sceller ledit deuxième sachet.

9. Procédé d'emballage selon la revendication 4 à 8 comportant en outre les étapes additionnelles consistant à :
- placer le deuxième sachet ainsi obtenu à l'intérieur d'un troisième sachet et
- sceller ledit troisième sachet.

10. Procédé d'emballage selon l'une des revendications 4 à 9, dans lequel lesdits deuxième et/ou troisième sachets sont constitués d'un matériau pris dans le groupe comprenant : la cellophane, les polyoléfines, les polyamides.

## Patentansprüche

1. Verwendung einer Polymerfolie zum Verpacken mindestens einer Petrischale, die ein Mikroorganismenkulturmedium in Agar-Form enthält, wobei die Folie mindestens eine Polystyrolschicht und mindestens eine Heißsiegelschicht umfasst, wobei die Folie eine mittlere Wasserdampfdurchlässigkeit zwischen 30,0 g/m² x 24 Stunden und 140,0 g/m² x 24 Stunden, vorzugsweise zwischen 70,0 g/m² x 24 Stunden und 120,0 g/m² x 24 Stunden aufweist, und wobei mindestens eine Polymerschicht mit Perforationen mit einer Größe zwischen 10 µm und 50 µm mikroperforiert ist.

2. Verwendung nach Anspruch 1, wobei die Folie eine Dicke zwischen 30 und 50 µm aufweist.

3. Verwendung einer Polymerfolie zur Herstellung eines Beutels zum Verpacken mindestens einer Petrischale, die ein Mikroorganismenkulturmedium in Agar-Form enthält, wobei die Folie mindestens eine Polystyrolschicht und mindestens eine Heißsiegelschicht umfasst, wobei die Folie eine mittlere Wasserdampfdurchlässigkeit zwischen 30,0 g/m² x 24 Stunden und 140,0 g/m² x 24 Stunden, vorzugsweise zwischen 70,0 g/m² x 24 Stunden und 120,0 g/m² x 24 Stunden aufweist, und wobei mindestens eine Polymerschicht mit Perforationen mit einer Größe zwischen 10 µm und 50 µm mikroperforiert ist.

4. Verfahren zum Verpacken mindestens eines Kulturmediums, umfassend die folgenden Schritte:
- Platzieren des Kulturmediums bzw. der Kulturmedien auf einer Folie, die mindestens eine Polystyrolschicht und mindestens eine Heißsiegelschicht umfasst, wobei die Folie eine mittlere Wasserdampfdurchlässigkeit zwischen 30,0 g/m² x 24 Stunden und 140,0 g/m² x 24 Stunden, vorzugsweise zwischen 70,0 g/m² x 24 Stunden und 120,0 g/m² x 24 Stunden aufweist, und mindestens eine Polymerschicht der Folie mit Perforationen mit einer Größe zwischen 10 µm und 50 µm mikroperforiert ist, wobei das Platzieren des Kulturmediums oder der Kulturmedien auf der Heißsiegelschicht der Folie durchgeführt wird;
- Bedecken des Kulturmediums bzw. der Kulturmedien mit einem frei gebliebenen Abschnitt der Folie oder mit einer anderen Folie, so dass die Heißsiegelschichten einander gegenüberliegen;
- Verbinden der Ränder der Folie oder der beiden Folien, so dass das Kulturmedium bzw. die Kulturmedien in dem so hergestellten Beutel eingeschlossen ist/sind.

5. Verpackungsverfahren nach dem vorhergehenden Anspruch, wobei die Folie(n) zuvor sterilisiert wird/werden.

6. Verpackungsverfahren nach dem vorhergehenden Anspruch, wobei es sich bei dem Sterilisationsverfahren um eine Bestrahlung mit Strahlen aus der Gruppe, bestehend aus gamma- und/oder beta-Strahlen, handelt.

7. Verpackungsverfahren nach einem der Ansprüche 4 bis 6, wobei der Verbindungsschritt ein Heißsiegelungsschritt bei einer Temperatur zwischen 100 und 170°C ist.

8. Verpackungsverfahren nach einem der Ansprüche 4 bis 7, das ferner die folgenden zusätzlichen Schritte umfasst:
- Platzieren des so erhaltenen Beutels im Inneren eines zweiten Beutels und
- Verschließen des zweiten Beutels.

9. Verpackungsverfahren nach Anspruch 4 bis 8, das ferner die folgenden zusätzlichen Schritte umfasst:
- Platzieren des so erhaltenen zweiten Beutels im Inneren eines dritten Beutels und
- Verschließen des dritten Beutels.

10. Verpackungsverfahren nach einem der Ansprüche 4 bis 9, wobei der zweite und/oder der dritte Beutel aus einem Material aus der Gruppe, umfassend: Zellophan, Polyolefine, Polyamide, bestehen.

## Claims

1. Use of a polymer film for packaging at least a Petri dish comprising one microorganism culture medium in an agar form, said film comprising at least one layer of polystyrene and at least one heat-sealing layer, said film having an average water vapour permeability of between 30.0 g/m² x 24 hours and 140.0 g/m² x 24 hours, preferentially between 70.0 g/m² x 24 hours and 120.0 g/m² x 24 hours, in which at least one layer is microperforated with perforations having sizes of between 10 µm and 50 µm.

2. Use according to Claim 1 , in which the film has a thickness of between 30 and 50 µm.

3. Use of a polymer film for producing a sachet intended for the packaging of at least a Petri dish comprising one microorganism culture medium in an agar form, said film comprising at least one layer of polystyrene and at least one heat-sealing layer, said film having an average water vapour permeability of between 30.0 g/m² x 24 hours and 140.0 g/m² x 24 hours, preferentially between 70.0 g/m2 x 24 hours and 120.0 g/m2 x 24 hours, in which at least one layer is microperforated with perforations having sizes of between 10 µm and 50 µm.

4. Process for packaging at least one culture medium, comprising the steps consisting in:
- placing the culture medium or media on a film film comprising at least one layer of polystyrene and at least one heat-sealing layer, said film having an average water vapour permeability of between 30.0 g/m² x 24 hours and 140.0 g/m² x 24 hours, preferentially between 70.0 g/m2 x 24 hours and 120.0 g/m2 x 24 hours, in which at least one layer is microperforated with perforations having sizes of between 10 µm and 50 µm, said placing of the culture medium or media being carried out on the heat-sealing layer of said film.
- covering the culture medium or media with a portion, which has remained free, of said film or with another film, such that the heat-sealing layers are facing one another,
- securing the edges of the film or of the two films, such that the culture medium or media is or are trapped in the sachet thus formed.

5. Packaging process according to the preceding claim, in which the film(s) is (are) presterilized.

6. Packaging process according to the preceding claim, in which the sterilization method is irradiation with radiation taken from the group consisting of gamma-rays and/or beta-rays.

7. Packaging process according to one of Claims 4 to 6, in which the securing step is a heat-sealing step at a temperature of between 100 and 170°C.

8. Packaging process according to one of Claims 4 to 7, also comprising the additional steps consisting in:
- placing the sachet thus obtained inside a second sachet and
- sealing said second sachet.

9. Packaging process according to one of Claims 4 to 8, also comprising the additional steps consisting in:
- placing the second sachet thus obtained inside a third sachet and
- sealing said third sachet.

10. Packaging process according to one of Claims 4 to 9, in which said second and/or third sachets consist of a material taken from the group comprising: cellophane, polyolefins and polyamides.
